# EUROPÄISCHE PATENTSCHRIFT

(11) **EP 0 585 765 B1**
(45) Veröffentlichungstag und Bekanntmachung des Hinweises auf die Patenterteilung: **07.01.1998**
(21) Anmeldenummer: 93113418.3
(22) Anmeldetag: 23.08.1993
(51) Int. Cl.: A61F 13/15

(54) **Wegwerfwindeln**
Disposable diaper
Couches à jeter

(30) Priorität: 25.08.1992 JP 225945/92
(43) Veröffentlichungstag der Anmeldung: 09.03.1994
(73) Patentinhaber: UNI-CHARM CORPORATION, Kawanoe-shi Ehime-ken (JP)
(72) Erfinder: Fujioka, Yoshihisa, Mitoyo-gun, Kagawa-ken (JP); Mukai, Hirotomo, Ehime-ken (JP)
(74) Vertreter: Sperling, Rüdiger, Dipl.-Ing.

(56) Entgegenhaltungen:
- EP-A- 0 422 504
- FR-A- 2 517 525
- US-A- 3 225 918
- US-A- 5 137 525

## Beschreibung

Die Erfindung betrifft
*eine Wegwerfwindel, bestehend aus einer flüssigkeitsdurchlässigen Decklage, einer atmungsfähigen, jedoch flüssigkeitsundurchlässigen Außenlage und einer zwischen diese gelegte flüssigkeitsabsorbierende Platte.*

Herkömmlicherweise wird für typische Wegwerfwindeln feuchtigkeitsdurchlässige, jedoch flüssigkeitsundurchlässige Folie, beispielsweise Kunststoffolie, gemischt mit feinen anorganischen Teilchen, als Außenlage verwendet, um ein unangenehmes Feuchtigkeitsgefühl, das oftmals von Windelträgern empfunden wird, vollständig zu vermeiden oder abzumildern.

Eine derartige Außenlage hat eine Feuchtigkeitsdurchlässigkeit im Bereich von 1500 bis 3000 g/m² 24h (ASTM E96-66) und einen Wasserdruckwiderstand von 1000 cm H₂O oder höher (JIS L 1092). Diese Werte haben sich als ausreichend für die Urinundurchlässigkeit, jedoch nicht als ausreichend für eine gewünschte Feuchtigkeitsdurchlässigkeit erwiesen. Daher kann die innerhalb der Windet erzeugte Feuchtigkeit durch eine derartige Außenlage nicht auf ein gewünschtes Niveau unterdrückt oder abgemildert werden, bei dem den Trägern kein unangenehmes Gefühl vermittelt wird und bei dem sie im wesentlichen frei von allen nachteiligen Auswirkungen von Feuchtigkeit sind, die möglicherweise eine Hautkrankheit verursachen kann. Je höher die Feuchtigkeitsdurchlässigkeit der Außenlage ist, desto niedriger sollte die Flüssigkeitsundurchlässigkeit derselben sein, was zum Austreten von flüssigen Ausscheidungen führt. Angesichts dieser widersprüchlichen Beziehung wird gewöhnlich der Flüssigkeitsundurchlässigkeit höchste Priorität verliehen.

Ein typisches Beispiel einer Wegwerfwindel des offenen Typs mit Bandbefestigungseinrichtungen, die zum Befestigen von Vorder- und Hinterteil aneinander in der Ebene der Hüftlinie verwendet werden, ist im japanischen Patent Nr. 1977-40267 aufgezeigt. Dieses Beispiel umfaßt eine flüssigkeitsdurchlässige Decklage, eine flüssigkeitsundurchlässige Außenlage und eine zwischen diese gelegte, flüssigkeitsabsorbierende Platte, wobei von Abschnitten der Deck- und der Außenlage, die sich nach außen seitlich über die einander seitlich gegenüberliegenden Seiten der Platte hinaus erstrecken, Seitenklappen gebildet werden und die jeweiligen Seitenklappen im Schrittbereich mit Ausschnitten versehen sind, die zur Bildung von Beinöffnungen bestimmt sind, um die die jeweiligen Seitenklappen mit elastischen Elementen versehen sind, die zum Abdichten der Seitenklappen um das jeweilige Bein des Trägers dienen, und wobei der Hinterteil an seitlich gegenüberliegenden Seiten mit Befestigungsbändern versehen ist, die zum Befestigen des Hinterteils am Vorderteil verwendet werden.

Bei der typischen, von vorstehend bezeichnetem japanischen Patent Nr. 1977-40267 aufgezeigten Windel verringern die in den entgegengesetzten Seiten des Schrittbereiches zur verbesserten Paßform der Windel am Körper des Trägers ausgebildeten Ausschnitte notwendigerweise die Breite des Schrittbereiches, und es ist praktisch unmöglich, daß der Schrittbereich die Oberschenkel des Trägers umgibt. Der auf diese Weise breitenreduzierte Schrittbereich verringert unvermeidlich die Fähigkeit des Schrittbereiches, flüssige Ausscheidungen zu absorbieren und feste Ausscheidungen festzuhalten, so daß das Austreten von Ausscheidungen, insbesondere flüssigen Ausscheidungen ohne weiteres entlang den einander gegenüberliegenden Seitenrändern des Schrittbereiches auftritt.

Allgemein verläuft in der bekannten Windel des in vorstehend bezeichnetem japanischen Patent aufgezeigten Typs eine Faltlinie des Schrittbereiches, die einer Grenzlinie des Vorder- und des Hinterteils entspricht, horizontal parallel zur Hüftlinie. Darüberhinaus hat die flüssigkeitsabsorbierende Platte sogenannte halbstarre Eigenschaften, da sie oftmals eine mehr oder weniger komprimierte Ansammlung von Faserpulpe und Seidenpapier, das die Ober- und Unterfläche dieser Ansammlung bedeckt, umfaßt. Demgemäß kann der Schrittbereich der Windel sich nicht dem entsprechenden Bereich des Körpers des Trägers anpassen, womit dem Träger nicht nur das Gefühl von mangelnder Paßform vermittelt wird, sondern auch der Flüssigkeitsaustritt verursacht wird.

Eine weitere Wegwerfwindel ist aus der gattungsbildenden EP-A-0 422 504 bekannt. Diese Windel weist am vorderen bzw. alternativ am rückseitigen Hüftbereich der Windel eine flüssigkeitsundurchlässige, jedoch atmungsfähige Außenlage auf.

Nachteilig an dieser Windel ist, daß die atmungsfähige Außenlage lediglich im Hüftbereich des Vorder- bzw. Hinterteils der Windel ausgebildet ist. Die atmungsfähige Außenlage wird verwendet, um die Feuchtigkeit in der Windel auf ein bestimmtes Niveau zu reduzieren, um dem Träger zum einen ein angenehmes Gefühl zu vermitteln und zum anderen die nachteiligen Auswirkungen von Feuchtigkeit, wie z.B. Hautkrankheiten zu verhindern. Ist die atmungsaktive Lage lediglich im Hüftbereich der Vorder- oder Rückseite der Windel ausgebildet, so ist die Fläche für einen wirksamen Feuchtigkeitsabbau gering. Hierbei muß beachtet werden, daß die Atmungsfähigkeit nur soweit ausgeprägt sein kann, daß eine ausreichende Flüssigkeitsundurchlässigkeit immer noch gewährleistet ist.

Ein weiterer Nachteil der in der EP-A-0 422 504 aufgezeigten Windel besteht darin, daß eine nur teilweise atmungsfähige Außenlage in der Herstellung sehr aufwendig ist.

Der Erfindung liegt daher die Aufgabe zugrunde, eine Windel zu schaffen, die bei einfacher Herstellung eine erhöhte Feuchtigkeitsdurchlässigkeit erlaubt, um negativen Auswirkungen wie Hautkrankheiten entgegenzuwirken.

Diese Aufgabe wird erfindungsgemäß dadurch gelöst, daß die Windel ein getrennt voneinander geformtes Vorder- und Hinterteil aufweist, wobei das Vorderteil und das Hinterteil entlang dem jeweiligen unteren Ende eines Schrittbereichs verschweißt sind und die Atmungsfähigkeit des Vorderteils und der des Hinterteils verschieden ist.

Durch die getrennte Herstellung von Vorderteil und Hinterteil und dem anschließenden Verschweißen entlang dem jeweiligen unteren Ende ist es möglich, auf eine sehr kostengünstige Art und Weise jeweils das gesamte Vorderteil oder - je nach Bedarf - auch das gesamte Hinterteil mit einer atmungsfähigen, jedoch flüssigkeitsundurchlässigen Außenlage zu versehen.

Vorzugsweise sind der Vorderteil und der Hinterteil nahe an unteren Enden entlang einer konvex in Richtung der Hüftlinie des Vorderteils und des Hinterteils gekrümmten Schweißlinie miteinander verschweißt, um so den Schrittbereich zu bilden.

Vorzugsweise sind der Vorderteil und der Hinterteil teilweise außerhalb des durch die konvex gekrümmte Schweißlinie gebildeten Schrittbereiches ausgeschnitten.

Es versteht sich, daß die Außenlage im Vorderteil oder im Hinterteil eine Atmungsfähigkeit hat, die niedriger ist als die der Außenlage im jeweils anderen Teil, die innerhalb des Umfangs der Erfindung überhaupt keine Atmungsfähigkeit haben kann.

Gemäß der Erfindung mit vorstehend umrissenem Aufbau wird ein Luftaustausch zwischen der Windel und der Außenluft durch den Vorderteil oder den Hinterteil erzielt, der die Außenlage mit höherer Atmungsfähigkeit einschließt.

Wird die Windel dem Träger angelegt, so ist die konvex gekrümmte Schweißlinie ordnungsgemäß im Schritt des Trägers positioniert und wenigstens die einander seitlich gegenüberliegenden Seiten des Schrittbereiches der Windel bedecken die Innenseiten der Oberschenkel des Trägers, womit der Schrittbereich der Windel im wesentlichen den gesamten Schritt des Trägers bedeckt.

### KURZBESCHREIBUNG DER FIGUREN

Die Erfindung wird anhand eines Beispiels unter Bezug auf die beiliegenden Figuren erläutert, wobei
- Fig. 1: eine Vorderansicht ist, die eine Ausführungsform einer gemäß der Lehre der Erfindung aufgebauten Wegwerfwindel zeigt; und
- Fig. 2: eine Schnittdarstellung entlang einer Linie X-X in Fig. 1 ist.

### BESCHREIBUNG DER BEVORZUGTEN AUSFÜHRUNGSFORM

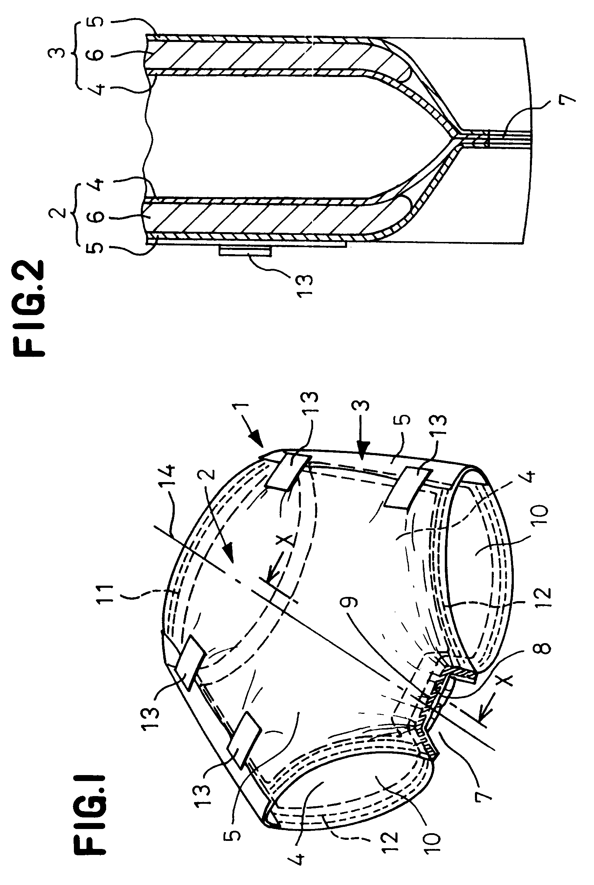

Wie in Fig. 1 und 2 gezeigt, umfaßt eine Windel 1 allgemein einen Vorderteil 2 und einen Hinterteil 3. Der Hinterteil 3 ist in Querrichtung größer dimensioniert als der Vorderteil 2. Der Vorderteil 2 und der Hinterteil 3 umfassen jeweils eine flüssigkeitsdurchlässige Decklage 4, eine flüssigkeitsundurchlässige Außenlage 5 und eine zwischen die Deck- und die Außenlage 4, 5 gelegte flüssigkeitsabsorbierende Platte 6.

Die unteren Enden des Vorderteils 2 und des Hinterteils 3 sind in mittleren Positionen mit konvex auf die Hüftlinien des Vorderteils 2 und des Hinterteils 3 zu gekrümmten Ausschnitten 7 versehen. Der Vorderteil 2 und der Hinterteil 3 sind entlang einer thermischen oder Ultraschall-Schweißlinie 8 miteinander verschweißt, die parallel zu den Ausschnitten 7 verläuft, so daß schmale Ränder der Ausschnitte 7 unverschweißt belassen sind. Größen, Formen und Krümmungsradien der Schweißlinie 8 können in Abhängigkeit davon, ob die Windel für einen Erwachsenen oder für ein Baby vorgesehen ist, in geeigneter Weise gewählt werden, sofern die Schweißlinie 8 konvex zur Hüftlinie des Vorderteils 2 und des Hinterteils 3 hin gekrümmt ist und wenigstens die Innenteile der jeweiligen Beinöffnungen 10 sich über einen Scheitelpunkt 9 der konvex gekrümmten Schweißlinie 8 hinaus nach unten erstrecken.

In Umfangsrichtung dehnbare elastische Elemente 11, 12 sind zwischen die Ränder der Deck- und der Außenlage 4, 5 gelegt, die sich über die Platte 6 hinaus um die Hüftöffnung und die jeweiligen Beinöffnungen erstrecken, und die Ränder sind durch Heißschmelzkleber oder durch Schweißeinrichtungen miteinander verbunden. Seitlich gegenüberliegende Seitenränder von Deck- und Außenlage 4, 5, die sich über die Platte 6 hinaus erstrecken, sind ebenfalls in derselben Weise miteinander verbunden, und der Hinterteil 3 ist an einander seitlich gegenüberliegenden Seitenrändern mit mehreren Befestigungseinrichtungen 13 versehen, die jeweils ein an einer Seite mit druckempfindlichem Klebstoff beschichtetes Befestigungsband umfassen, durch das die einander seitlich gegenüberliegenden Seiten des Hinterteils 3 an den entsprechenden Seiten des Vorderteils 2 befestigt werden.

Während die einander seitlich gegenüberliegenden Seitenränder und unteren Ränder um die jeweiligen Beinöffnungen des Vorderteils 2 und des Hinterteils 3 so dargestellt sind, daß sie weder parallel noch senkrecht zu einer vertikalen Achse 14 verlaufen, können innerhalb des Umfanges der Erfindung diese Seitenränder parallel zur vertikalen Achse 14 und diese unteren Ränder um die jeweiligen Beinöffnungen senkrecht zur vertikalen Achse 14 verlaufen.

Die Windel 1 ist an den einander seitlich gegenüberliegenden Seiten offen und daher sowohl an der Hüftöffnung als auch an den Beinöffnungen offen, und nach dem Aufrichten unter Verwendung der Befestigungseinrichtungen, wie in Fig. 1 dargestellt, sind die einander seitlich gegenüberliegenden Seiten geschlossen und folglich sind die Hüftöffnung wie auch die Beinöffnungen ebenfalls geschlossen. Die Größen der Öffnungen sind von der Überlappungsbreite des Vorder- und des Hinterteils 2, 3 abhängig und die Überlappungsbreite wiederum hängt von der Größe der Hüfte und der Beine (Oberschenkel) des einzelnen Trägers ab. Es ist auch möglich, die einander seitlich gegenüberliegenden Seitenränder miteinander zu verschweißen, um eine Windel des Höschentyps zu erhalten, wobei in diesem Fall keine Befestigungseinrichtungen erforderlich sind.

Der Vorderteil 2 oder der Hinterteil 3 verwenden die Außenlage 5 mit einer Atmungsfähigkeit von 3 cc/cm²/sek. oder höher und einem Wasserdruckwiderstand von 50 cm H₂O oder höher. Ob der Vorderteil 2 oder der Hinterteil 3 die Außenlage mit einer derartigen Atmungsfähigkeit und einem derartigen Wasserdruckwiderstand verwenden sollten, ist beispielsweise davon abhängig, ob die Windel für einen Erwachsenen oder für ein Baby vorgesehen ist. Allgemein werden erwachsene Patienten auf dem Rücken liegen und weder Körpergewicht noch eine nennenswerte Wasserdruckwiderstandslast wirkt auf den Vorderteil der Windel, so daß die Außenlage mit einer höheren Atmungsfähigkeit im Vorderteil verwendet werden kann, um die möglicherweise innerhalb der Windel entstehende Feuchtigkeit möglichst gering zu halten oder abzumildern. Andererseits liegen einige Babies mit dem Gesicht nach oben und einige Babies mit dem Gesicht nach unten. Im letzteren Fall kann die Außenlage mit höherer Atmungsfähigkeit im Hinterteil verwendet werden.

Als Material zur Herstellung der Außenlage mit höherer Atmungsfähigkeit wird bevorzugt hydrophober Vliesstoff mit relativ hoher Dichte, wasserabweisend behandelter Vliesstoff, mit einer Vielzahl von feinen Nadelstichen versehene Kunststoffolie, feinmaschiges Netz oder ähnliches verwendet.

Die anderen Bestandteile der Windel 1 können den allgemein für die bekannte Windel verwendeten entsprechen. Beispielsweise kann die Decklage 4 aus Vliesstoff, die Außenlage 5, abgesehen von der hoch atmungsfähigen Außenlage, aus Kunststoffolie, die Platte 6 aus Faserpulpe, gemischt mit hochabsorbierendem Polymer hergestellt sein, die elastischen Elemente 11, 12 können aus natürlichem oder synthetischem Gummi, und der Trägerstoff der Befestigungseinrichtung 13 kann aus qualitativ hochwertigem Papier oder einem Schichtstoff aus Vliesstoff und Kunststoffolie hergestellt sein.

Die gemäß der Erfindung wie vorstehend beschrieben aufgebaute Windel erlaubt es, unerwünschte Feuchtigkeit, die mehr oder weniger innerhalb der Windel nach dem Stand der Technik entstand, möglichst gering zu halten oder abzumildern, indem die Atmungsfähigkeit eines Vorderteils oder eines Hinterteils in Abhängigkeit von der Art des Endproduktes selektiv höher eingestellt wird als die des anderen Teils, und erlaubt es gleichzeitig, häufig bei der herkömmlichen Windel auftretendes Austreten von Ausscheidungen in wirksamer Weise durch den anderen Teil mit niedrigerer Atmungsfähigkeit zu vermeiden.

Gemäß der Erfindung führt die Ausbildung von Ausschnitten an entgegengesetzten Seiten des Schrittbereiches, um so die Beinöffnungen zu bilden, niemals zu einer inakzeptabel schmalen Breite des Schrittbereiches, da die konvex auf die Hüftlinie zu gekrümmte Schweißlinie es erlaubt, daß die zwischen den gegenüberliegenden Seitenrändern des Schrittbereiches bestimmte Breite des Schrittbereiches angemessen groß dimensioniert ist. In dieser Weise bedeckt der Schrittbereich wenigstens die Innenseiten der jeweiligen Oberschenkel ausreichend, um seine Absorptionsfähigkeit zu verbessern und dadurch das Austreten von Ausscheidungen entlang seinen Rändern um die jeweiligen Oberschenkel zu verhindern. Insbesondere bei einer Windel des offenen Typs ermöglicht das Vorsehen dieser konvex gekrümmten Schweißlinie, die Windel ordnungsgemäß dem Träger anzulegen, da der Schrittbereich der Windel sich dem entsprechenden Bereich des Körpers des Trägers gut anpaßt.

## Patentansprüche

1. Wegwerfwindel, bestehend aus einer flüssigkeitsdurchlässigen Decklage (4), einer atmungsfähigen, jedoch flüssigkeitsundurchlässigen Außenlage (5) und einer zwischen diese gelegte flüssigkeitsabsorbierende Platte (6), **dadurch gekennzeichnet**, daß die Wegwerfwindel einen getrennt voneinander geformten Vorderteil (2) und Hinterteil (3) aufweist, wobei der Vorderteil (2) und der Hinterteil (3) miteinander, entlang dem jeweiligen unteren Ende eines Schrittbereiches (8) verschweißt sind und die Atmungsfähigkeit des Vorderteils von der des Hinterteils verschieden ist.

2. Wegwerfwindel nach Anspruch 1,
wobei der Vorderteil (2) und der Hinterteil (3) nahe an unteren Enden entlang einer konvex in Richtung der Hüftlinie des Vorderteils und des Hinterteils gekrümmten Schweißlinie (8) miteinander verschweißt sind, um so den Schrittbereich zu bilden.

3. Wegwerfwindel nach Anspruch 1,
wobei der Vorderteil (2) und der Hinterteil (3) teilweise außerhalb des durch die konvex gekrümmte Schweißlinie gebildeten Schrittbereiches ausgeschnitten sind.

## Claims

1. A disposable nappy, consisting of a cover sheet (4) permeable to liquid, an outer sheet (5) which is impermeable to liquid but which can breath, and a liquid-absorbing layer (6) placed therebetween, characterized in that the disposable nappy has separately formed front and rear parts (2 and 3), wherein the front part (2) and the rear part (3) are welded together along the respective lower ends of a crotch region (8) and the ability to breath of the front part is different from that of the rear part.

2. A disposable nappy according to claim 1, wherein the front part (2) and the rear part (3) are welded together near to the lower ends along a weld line (8) curved convexly in the direction of the hip line of the front part and the rear part, in order thus to form the crotch region.

3. A disposable nappy according to claim 1, wherein the front part (2) and the rear part (3) are partially cut out outside the crotch region formed by the convexly curved weld line.

## Revendications

1. Lange à jeter, constitué d'une couche de revêtement (4) perméable aux liquides, d'une couche extérieure (5) aérée, mais imperméable aux liquides, et d'une plaque (6) absorbant les liquides, insérée entre ces deux couches, caractérisé en ce que le lange à jeter présente une partie avant (2) et une partie arrière (3) formées séparément l'une de l'autre, la partie avant (2) et la partie arrière (3) étant soudées entre elles en suivant l'extrémité inférieure respective d'une zone d'entrejambe (8), et l'aération de la partie avant étant différente de celle de la partie arrière.

2. Lange à jeter suivant la revendication 1, dans lequel la partie avant (2) et la partie arrière (3) sont soudées entre elles à proximité des extrémités inférieures, en suivant une ligne de soudure (8) qui s'incurve selon un tracé convexe dans la direction de la ligne de hanche de la partie avant et de la partie arrière, pour former ainsi l'entrejambe.

3. Lange à jeter suivant la revendication 1, dans lequel la partie avant (2) et la partie arrière (3) sont découpées en partie à l'extérieur de la zone d'entrejambe formée par la ligne de soudure à courbure convexe.
